# EUROPÄISCHE PATENTSCHRIFT

(11) **EP 0 532 915 B1**
(45) Veröffentlichungstag und Bekanntmachung des Hinweises auf die Patenterteilung: **08.01.1997**
(21) Anmeldenummer: 92113897.0
(22) Anmeldetag: 14.08.1992
(51) Int. Cl.: C07K 9/00, C12P 19/26, A61K 38/00, A61K 31/715

(54) **Phosphoinositolglykan-Peptid mit insulinartiger Wirkung**
Phosphoinositolglycan-peptide with insulin like activity
Phosphoinositolglycan-peptide à activité de type insuline

(30) Priorität: 20.08.1991 DE 4127495
(43) Veröffentlichungstag der Anmeldung: 24.03.1993
(73) Patentinhaber: HOECHST AKTIENGESELLSCHAFT, 65926 Frankfurt am Main (DE)
(72) Erfinder: Müller, Günter, Dr., W-6231 Sulzbach/Taunus (DE); Tripier, Dominique, Dr., W-6239 Eppstein/Taunus (DE); Müllner, Stefan, Dr., W-6203 Hochheim (DE)

(56) Entgegenhaltungen:
- EP-A- 0 245 956
- BIOCHEMICAL AND BIOPHYSICAL RESEARCH COMMUNICATIONS. Bd. 166, Nr. 2 , 30. Januar 1990 , DULUTH, MINNESOTA US Seiten 765 - 771 P. BRUNI ET AL 'A phospho-oligosaccharide can reproduce the stimulatory effect of insulin on glycolytic flux in human fibroblasts'
- NATO ASI SERIES Bd. H44 , 1990 , HEIDELBERG Seiten 167 - 179 I. VALERA ET AL 'Role of glycosyl-phosphatidylinositols in insulin signalling'
- BIOCHEMICAL AND BIOPHYSICAL RESEARCH COMMUNICATIONS. Bd. 164, Nr. 2 , 31. Oktober 1989 , DULUTH, MINNESOTA US Seiten 824 - 832 M. LISANTI ET AL 'The distribution of glycosyl-phosphatidylinositol anchored proteins is differential regulated by serum and insulin'
- BIOLOGICAL CHEMISTRY HOPPE-SEYLER Bd. 372, Nr. 9 , September 1991 Seiten 718 - 719 G. MUELLER AND W. BANDLOW 'Insulin causes lipolytic cleavage and endocytosis of a cAMP-binding protein from plasma membranes in rat adipocytes'
- BIOLOGICAL ABSTRACTS, xol. 93, no. 2 15. Januar 1992, Philadelphia, PA, US; abstract no. 14568, G. MUELLER AND W. BANDLOW 'A cAMP-binding ectoprotein in the yeast Saccharomyces cerevisiae' Seite 242 ;

## Beschreibung

Die Erfindung betrifft Phosphoinositolglykan-Peptide mit insulinartiger Wirkung, Verfahren zur Herstellung derselben und Verwendung, insbesondere als Arzneimittel zur Behandlung von Diabetes mellitus oder nicht insulinabhängiger Diabetes.

Insulin übt eine Vielzahl von Wirkungen auf Insulin-Sensitives Gewebe aus. Ein auffälliger Effekt ist die schnelle Reduktion des Glucosespiegels in Säugetieren, wenn Insulin angewendet wird. Dies wird durch eine schnelle Aufnahme der Glucose aus dem Blut durch Muskel- und Fettzellen bewirkt. Insulin aktiviert ferner die Glycogen-Synthetase und inhibiert die Lipolyse. Insulin fördert die Proteinsynthese aus Aminosäuren und verstärkt die Induktion von Pyruvatdehydrogenase und Phosphofructokinase und inhibiert die Bildung von bestimmten Enzymen der Gluconeogenese, wie Pyruvatcarboxylase und Fructose-1,6-bisphosphatase.

Der Diabetes Typ II, nicht insulinabhängiger Diabetes, geht einher mit einer Insulin-Resistenz der peripheren Gewebe wie Muskel- oder Fettgewebe. Die dadurch reduzierte Glukoseverwertung wird verursacht durch fehlende Insulin-Stimulation des Glucosetransports und nachfolgender metabolischer Prozesse (Glucogenese, Lipogenese). Diese multiple Resistenz läßt auf einen Defekt auf Rezeptor- oder Post-Rezeptor-Ebene, d. h. vor der Erzeugung der "second messenger", schließen (Garvey, Diabetes/Metabolism Reviews, 5, (1989), 727 - 742).

Bisher sind keine Wirkstoffe bekannt, die den Insulinrezeptor umgehen und trotzdem eine insulinartige Wirkung zeigen.

Es wurde nun gefunden, daß Phosphoinositolglykan-Peptide, die aus Adenosin-3',5'-cyclischen-monophosphat (cAMP)-Bindeprotein gewonnen werden können, in vitro insulinartige Wirkung zeigen und auch an Insulin-resistentem Gewebe insulinartige Wirkung zeigen.

Die Erfindung betrifft daher Phosphoinostolglykan-Peptid, dadurch gekennzeichnet, daß es Phosphoinositolglykan, Ethanolamin, Tripeptid und/oder physiologisch verträgliche Salze vom Phosphoinositolglykan-Peptid enthält und insulinartige Wirkung besitzt.

Unter dem Begriff insulin-resistentes Gewebe werden beispielsweise Ratten-Fettzellen verstanden, die keinen Insulinrezeptor mehr besitzen. cAMP-Bindeprotein sind Proteine, die die Eigenschaft besitzen, Adenosin-3',5'-cyclischenmonophosphat zu binden.

Die bisherigen Strukturdaten des erfindungsgemäßen Phosphoinositolglykan-Peptides weisen auf ein Phosphoinositol mit einem Glykananteil, der Galaktose, Glucosamin und Mannose enthält, hin, welches über einen Phosphorylethanolaminrest mit einer Amidbindung an das Carboxylende eines Peptids gekoppelt ist. Dieses findet sich innerhalb der als Membrananker dienenden kovalent gebundenen Glykolipide in ähnlicher Struktur in glypiierten Plasmamembranproteinen höherer Eukaryonten wieder (Roberts et al., J. Biol. Chem, 263, (1983), 18776 - 18784). Der Glykanrest enthält Glukosamin, Galaktose, Mannose und mindestens zwei Phosphatreste/Mol. Das Peptid besitzt die Sequenz -Asn-Cys-Tyr- und ist mit dem Carboxylende der Asparaginsäure über eine Amidbindung an die Aminogruppe des Phosphoinositolglykans gebunden. Der Phosphoinositolglykananteil ist für die Aktivität erforderlich. Das Peptid verstärkt die insulinartige Wirkung. Ferner zeigt sich, daß nicht nur das vollständige Phosphoinositolglykan-Peptid insulinartige Wirkung zeigt, sondern auch Teilstrukturen, beispielsweise zeigen auch verkürzte Peptidsequenzen, die am Phosphoinositolglykan gebunden sind insulinartige Wirkung oder nur das Phosphoinositolglykan ohne Peptid.

Geeignete physiologisch verträgliche Salze von dem erfindungsgemäßen Phosphoinositolglykan-Peptid sind beispielsweise Alkali-, Erdalkali- oder Ammoniumsalze, sowie solche von physiologisch verträglichen, organischen Ammonium- oder Triethylaminbasen.

Die Herstellung der erfindungsgemäßen Phosphoinositolglykan-Peptide erfolgt beispielsweise dadurch, daß man
a) Organismen, die cAMP-Bindeprotein enthalten, verwendet,
b) das cAMP-Bindeprotein isoliert,
c) den Proteinanteil von cAMP-Bindeprotein abspaltet und das entstandene Glycosyl-Phosphatidylinositol-Peptid gegebenenfalls reinigt,
d) von dem im Verfahrensschritt c) erhaltenen Produkt ein Mono- oder Diacylglycerin abspaltet und
e) das in Verfahrensschritt d) entstandene Phosphoinositolglykan-Peptid isoliert.

cAMP-Bindeprotein kann aus zahlreichen Organismen gewonnen werden, beispielsweise aus Mikroorganismen, Pflanzen, Pilzen oder tierischen Organen. Geeignet ist beispielsweise auch die Hefe Saccharomyces cerevisiae, insbesondere DSM 6649.

Die Herstellung des cAMP-Bindesproteins durch Saccharomyces cerevisiae erfolgt durch Fermentation in einer Nährlösung, die eine Kohlenstoff- und eine Stickstoffquelle, sowie übliche anorganische Salze enthält. Das cAMP-Bindeprotein wird bevorzugt in der Plasmamembran der Hefe angereichert.

Beim Verfahrensschritt a) geht man am besten wie folgt vor. Die Bildung von cAMP-Bindeprotein in Saccharomyces cerevisiae verläuft gut in den üblichen Nährlösungen für Saccharomyces cerevisae. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35 °C, vorzugsweise bei etwa 25 bis 30 °C, insbesondere 28 bis 30 °C durchgeführt werden. Der pH-Bereich sollte zwischen 2 und 8 liegen, vorteilhaft zwischen 3 und 7. Man kultiviert die Hefe unter diesen Bedingungen im allgemeinen bis zu etwa 10⁷ Zellen/ml Nährlösung.

Beim Verfahrensschritt b) geht man am besten so vor, daß die Hefezellen zur Isolierung des cAMP-Bindeproteins vom Nährmedium getrennt und mit Puffer gewaschen werden. Die Isolierung erfolgt beispielsweise wie bei Müller und Bandlow (Biochemistry, 28, (1989), 9957 - 9967) beschrieben. Dazu werden die Hefezellen enzymatisch (Zymolyase) in Spheroplasten umgewandelt und mit einem Homogenisator in Anwesenheit von Proteaseinhibitoren in der Kälte (0 - 4 °C) zerkleinert. Hefelysate werden zentrifugiert und der Zellniederschlag wird mit Puffer gewaschen und erneut zentrifugiert. Die Überstände werden vereinigt und in einem ®Percoll-Gradienten (28 % Percoll) und Saccharosegradienten (15 bis 28 % Saccharose) gereinigt. Durch diese Zentrifugationsschritte werden Cytoplasma, Plasmamembran, Microsomen und Mitochondrien voneinander getrennt.

Aus der Plasmamembranfraktion wird das cAMP-Bindeprotein z. B. durch eine N⁶-(2-Aminoethyl)-cAMP-Sepharose-Säule gebunden und das cAMP-Bindeprotein wird mit cAMP von der Säule eluiert und entsalzt.

Beim Verfahrensschritt c) geht man am besten so vor, daß der Proteinanteil des cAMP-Bindeproteins enzymatisch abgespalten wird. Zur enzymatischen Spaltung verwendet man beispielsweise Proteasen, wie Pronase, Endoprotease Lys-C (Lysobacter enzymogenes) oder V8 Protease (Staphylococcus aureus). Damit erreicht man den Abbau des Proteinanteils. Mit der Protease V8 erhält man ein Glycosyl-phosphatidylinositol mit der Peptidsequenz Asn-Cys-Tyr; Inkubation mit der Protease Pronase ergibt ein Glycosyl-phosphatidylinositol-Peptid, wobei der Peptidanteil nur aus der Aminosäure Asn besteht.

Die Protease wird beispielsweise durch Säuren wie Trichloressigsäure ausgefällt Die Glycosyl-Phosphatidylinositol-Peptide werden durch Zentrifugation von den Proteasen getrennt, mit einer Phenylsepharose-Säule konzentriert und mit Dünnschichtchromatographie gereinigt.

Beim Verfahrensschritt d) geht man am besten so vor, daß der am Glycosylphosphatidylinositol-Peptid hängende Mono- oder Diacylglycerinrest enzymatisch abgespalten und das Phosphoinositolglykan-Peptid dadurch freigesetzt wird. Zur enzymatischen Spaltung verwendet man beispielsweise Phospholipasen wie Phophatidylinositol spezifische Phospholipase C (Bacillus cereus). Damit erreicht man die Abspaltung des Mono- oder Diacylglycerinrestes, der über die Phosphatgruppe an das Myo-Inositol gebunden ist. Der glycerinhaltige Rest wird durch die Proteasebehandlung im Verfahrensschritt c) nicht abgespalten. Die Verfahrensschritte c) und d) können auch in umgekehrter Reihenfolge durchgeführt werden.

Die enzymatischen Reaktionen in den Verfahrensschritten c) und d) werden in Gegenwart von Detergenzien und unter Bedingungen durchgeführt wie sie für die enzymatische Reaktion günstig sind. Aufgrund der bekannten Reaktionsbedingungen für die genannten Enzyme, fällt es einem Fachmann nicht schwer die optimalen Reaktionsbedingungen zu ermitteln.

Beim Verfahrensschritt e) geht man am besten so vor, daß die Phospholipasen durch Säuren wie Trichloressigsäure ausgefällt werden. Das Phosphoinositolglykan-Peptid wird durch Zentrifugation von der Phospholipase getrennt, mit einer Biogel P-4 Säule gereinigt und durch Dünnschichtelektrophorese konzentriert.

Die erfindungsgemäßen Phosphoinositolglykan-Peptide und deren physiologisch verträglichen Salze dienen in erster Linie als Wirkstoffe für pharmazeutische Zubereitungen zur Behandlung des Diabetes mellitus oder nicht insulinabhängiger Diabetes.

Erfindungsgegenstand ist daher auch ein Arzneimittel, das durch einen wirksamen Gehalt an Phosphoinositolglykan-Peptid und/oder mindestens einem von dessen physiologisch verträglichen Salzen in gelöster, amorpher und/oder kristalliner Form gekennzeichnet ist.

Das Arzneimittel ist vorzugsweise eine Lösung oder Suspension zu Injektionszwecken mit einem pH-Wert zwischen etwa 3,0 und 9,0, vorzugsweise zwischen 5,0 und 8,5 welche ein geeignetes Isotoniemittel, ein geeignetes Konservierungsmittel und gegebenenfalls einen geeigneten Puffer, sowie gegebenenfalls auch ein Depotprinzip, alles natürlich in steriler wäßriger Lösung oder Suspension, enthält. Die Gesamtheit der Zubereitungsbestandteile außer dem Wirkstoff bildet den Zubereitungsträger.

Geeignete Isotoniemittel sind z. B. Glycerin, Glukose, Mannit, NaCl, Calcium- oder Magnesium-Verbindungen wie z. B. CaCl₂ oder MgCl₂.

Geeignete Konservierungsmittel sind z. B. Phenol, m-Cresol, Benzylalkohol und/oder p-Hydroxybenzoesäureester.

Als Puffersubstanzen, insbesondere zur Einstellung eines pH-Wertes zwischen etwa 5,0 und 8,5 können z. B. Natriumacetat, Natriumcitrat oder Natriumphosphat verwendet werden. Ansonsten sind zur Einstellung des pH-Wertes auch physiologisch unbedenkliche verdünnte Säuren (typischerweise HCl) bzw. Laugen (typischerweise NaOH) geeignet.

Zwecks Variation des Wirkungsprofils der erfindungsgemäßen Arzneimittel können auch modifizierte (vgl. EP-B 132 769 und EP-B 132 770) und/oder unmodifizierte Insuline, vorzugsweise Rinder-, Schweine- oder Humaninsulin, insbesondere Humaninsulin, zugemischt werden.

Das Arzneimittel wird dadurch hergestellt, daß man das Phosphoinositolglykan-Peptid und/oder mindestens eines von dessen physiologisch verträglichen Salzen, gegebenenfalls zusammen mit modifizierten und/oder unmodifizierten Insulin(derivat)en, mit einem physiologisch unbedenklichen Träger sowie gegebenenfalls mit geeigneten Zusatz- und Hilfsstoffen in eine geeignete Darreichungsform bringt.

Die Erfindung wird nun durch die folgenden Beispiele näher erläutert.

### Beispiel 1

### Fermentation von Saccharomyces cerevisiae DSM 6649

Saccharomyces cerevisiae DSM wird aerob in einem Fermenter kultiviert. Folgende Mediumsbestandteile sind in einem Liter Wasser enthalten:

| | |
|---|---|
| Hefeextrakt | 3 g |
| Glucose | 1 g |
| KH₂PO₄ | 1 g |
| NH₄Cl | 1 g |
| CaCl₂·2H₂O | 0,5 g |
| NaCl | 0,5 g |
| MgSO₄·H₂O | 0,6 g |
| FeCl₃ | 0,3 ml einer 1% wäßrigen Lösung |
| Milchsäure | 22 ml einer 90 %-Lösung |

Der pH wird mit KOH auf 5,5 eingestellt. Die Belüftung erfolgt mit Luft 1 l/l Fermentervolumen. Die Zellen werden bis zu einer Zelldichte von 1 x 10⁷ Zellen/ml Kulturlösung angezogen; Ausbeute etwa 3 g Feuchtgewicht pro I Kulturlösung. Die Zellen werden durch Zentrifugation geerntet (5 min 3000 x g) und mit Phosphatpuffer, pH 6, gewaschen.

### Beispiel 2

### Isolierung von cAMP-Bindeprotein

Die Zellen aus Beispiel 2 werden enzymatisch (Zymolyase, 2000, Seikagaku Kogyolo, Tokio) in Spheroplasten umgewandelt, mit einem Glashomogenisator (Arthur H. Thomas and Co.) bei 0 °C zerkleinert. Die folgenden Isolationsschritte erfolgen in Gegenwart von Proteaseinhibitoren (Phenylmethansulfonylfluorid (PMSF), Leupeptin, Aprotinin, α₂-Makroglobulin, Trypsin-Inhibitor; Boehringer Mannheim). Das Zellysat wird zentrifugiert (1000 x g, 3 min, 4 °C), das Zellsediment wird mit SEM-Puffer (0,25 M Saccharose, 0,5 mM Ethylendiamintetraessigsäure (EDTA), 20 mM 3-[N-morpholino]-propansulfonsäure (MOPS)/KOH, pH 7,4) gewaschen und erneut zentrifugiert. Die Überstände werden vereinigt und in einem ®Percoll-Gradienten (Pharmacia, Freiburg; 28 % Percoll, SEM-Puffer, 0,5 mg Protein/ml) zentrifugiert (18000 x g, 15 min, 4 °C). In diesem Gradienten werden Cytoplasma, Plasmamembran, Microsomen und Mitochondrien voneinander getrennt. Die Plasmamembranen flotieren im oberen Drittel des Gradienten. Sie werden mit einer Spritze vom Gradienten entnommen, mit 5 fachen Volumen des SEM-Puffers verdünnt und zentrifugiert (48000 x g, 30 min, 4 °C). Das Sediment wird in MOPS-Puffer (5 mg Protein/ml) suspendiert und mit N-[³H]Acetyl-Concanavalin A (Amersham Buchler, Braunschweig; 1 mg Protein mit 55 µCi, in 500 µl MOPS-Puffer (Boehringer Mannheim), 20 mM, pH 7,4, 0,5 mM EDTA, 50 mM KCl, 5 mM CaCl₂, 200 µg Rinderserumalbumin; Behring Werke, Marburg) im Ultraschallbad für 60 Minuten, 4 °C, inkubiert. Die Bindung von Concanavalin A dient als Marker. Die Suspensionen werden auf einen Saccharose-Gradienten (15 bis 28 % Saccharose in MOPS-Puffer) pipettiert und zentrifugiert (25000 Umdrehungen pro Minute, 90 min, 4 °C, Beckmann SW 27 Rotor). Die Saccharosegradienten werden fraktioniert und die radioaktiven Fraktionen (etwa 23 % Saccharose) werden vereinigt, 3 fach verdünnt mit einem MOPS-Puffer (50 mM Concanavalin A; Sigma Deisenhofen, 250 mM KCl) und zentrifugiert (200000 x g, 60 min, 4 °C, Beckmann TL-100-Rotor). Das Sediment wird mit SEM-Puffer (250 mM KCl) gewaschen, zentrifugiert und im SEM-Puffer ohne KCl resuspendiert (2,5 mg Protein/ml).

Etwa 500 µg der Plasmamembranproteine werden im Puffer solubilisiert (25 mM MOPS/KOH, pH 7,0, 150 mM NaCl, 4 mM MgCl₂, 0,4 mM EGTA (Ethylenglykol-bis(β-Aminoethylether)tetraessigsäure), 0,5 mM DTT (Dithiothreitol), 0,5 % Desoxycholat, 0,1 mM IBMX (Isobutylmethylxanthin), 0,1 mM PMSF, 50 µM Leupeptin, 0,1 mM Aprotinin (2 mg/ml). Die Lösung wird auf eine 2 ml N⁶-(2-Aminoethyl)-cAMP-Sepharose-Säule (Pharmacia, Freiburg) bei 4 °C aufgetragen, die mit dem gleichen Puffer equilibriert wurde. Die Säule wird fünfmal mit je 2 ml Puffer (25 mM MOPS/KOH, pH 7,2, 100 mM Na-Citrat, 5 mM DTT, 5 mM MgCl₂, 150 mM NaCl, 250 mM Saccharose, 7,5 % Ethylenglycoll (Merck, Darmstadt), 10 % Glycerin, 1 mg/ml Rinderserumalbumin, 1 mM IBMX) gewaschen. Eluiert wird bei 4 °C mit 2 ml des gleichen Puffers der zusätzlich 100 µM cAMP enthält. Die ersten 250 µl des Eluats werden durch Zentrifugation einer 1 ml Sephadex G-25-Säule entsalzt, die mit einem Puffer (25 mM MOPS/KOH, pH 7,0, 50 mM KCl, 5 mM MgCl₂, 10 mM DTT, 50 µM EDTA, 50 µM PMSF, 0,1 % Desoxycholat, 5 % Glycerin) equilibriert war. Das entsalzte Material wird mit dem gleichen Volumen von 8 % Polyethylenglycol 4000 (Pharmacia, Freiburg) in Puffer (10 mM MOPS/KOH, pH 7,2, 1 mM EDTA) für 30 Minuten bei 4 °C inkubiert. Nach 15 Minuten Zentrifugation wird das Sediment in einem Puffer (20 mM MOPS/KOH, pH 7,2, 1 mM EDTA, 100 µM PMSF, 0,5 % Desoxycholat) gelöst (2 mg Protein/ml).

### Beispiel 3

### Herstellung von Phosphoglykan-Peptiden

a) Verdau mit Pronase (Streptomyces griseus; Boehringer Mannheim): 100 µg cAMP-Bindeprotein wird für 10 Stunden bei 50 °C in 1 ml 0,1 M 4-(2-Hydroxyethyl)-1-piperazinethansulfonsäure (HEPES)/KOH (pH 8,0), 15 mM CaCl₂, 1 % Triton X-100 (Boehringer Mannheim) mit 450 µg/ml Pronase inkubiert. Nach Zugabe von 1 % SDS (Natriumdodecylsulfat) wird die Inkubation mit einem zweiten Aliquot Pronase für 7 h bei 50 °C fortgesetzt. Automatisierter Edmann-Abbau zeigt, daß der Pronaseabbau zu Phosphoinositolglykan mit am Aminoende des Ethanolamins amidartig gebundenem Asparagin führt. Dieses Phosphoinositolglykan-Peptid wird im folgenden mit PIG-N bezeichnet.
b) Verdau mit Endoproteinase Glu-C, EC 3.4.21.19, (V8 Protease) (Staphylococcus aureus; Boehringer Mannheim): 100 µg cAMP-Bindeprotein wird für 18 Stunden bei 37 °C in 1 ml 20 mM (NH₄)₂CO₃ (pH 7,8), 0,5 % Oktylglukosid (Boehringer Mannheim) mit 300 µg V8 Protease inkubiert.
   Automatisierter Edmann-Abbau zeigt, daß die V8 Protease-Verdauung zu dem Phosphoinositolglykan mit dem Peptid Asn-Cys-Tyr führt. Die Aminosäure Asparaginsäure ist mit dem Carboxyterminus an das Phosphoinositolglykan gebunden. Die Verbindung Phosphoinositolglykan mit dem Tripeptid Asn-Cys-Tyr wird im folgenden mit PIG-NCY bezeichnet.
c) Verdau mit Endoprotease Lys-C (Lysobacter enzymogenes; Boehringer Mannheim): 100 µg cAMP-Bindeprotein wird für 18 Stunden bei 37 °C in 0,5 ml 50 mM (NH₄)₂CO₃ (pH 8,2), 0,5 % Oktylglukosid mit 55 µg Endoprotease Lys-C inkubiert.
   Automatisierter Edmann-Abbau zeigt, daß die Endoprotease Lys-C zu dem Phosphoinositolglykan mit dem Peptid Asn-Cys-Tyr-Glu führt. Die Bindung des Peptids an das Phosphoinositolglykan erfolgt über das Carboxyende von Asparagin. Sie wird im folgenden mit PIG-NCYE bezeichnet.
d) Entfernung der carboxy-terminalen Aminosäure: Pronase-verdautes cAMP-Bindeprotein (siehe a) wird einem manuellen Edmann-Abbau unterzogen. Das entstandene Aminosäure-freie Phosphoinositolglykan wird im folgenden mit PIG bezeichnet.
   Nach den proteolytischen Spaltungen werden die Proteasen durch Präzipitation mit 5 % Trichloressigsäure (TCA) entfernt. Nach Zentrifugation (15 min, 10000 x g) werden die im Überstand enthaltenen Phosphoinositolglykan-Peptidderivate wie auch das Phosphoinositolglykan des Edmann-Abbaus durch Bindung an eine Phenylsepharose-Säule konzentriert und gereinigt. Nach Elution mit 2 % Octylphenol(ethylenglycolether) (TX-100) werden die Phosphoinositolglykan-Peptidderivate durch Dünnschichtchromatographie mit zwei verschiedenen Lösungsmittelsystemen gereinigt. Nach dem ersten Lauf in einem sauren System (Chloroform/Aceton/Methanol/Eisessig/Wasser 10 : 4 : 2 : 2: 1) werden die Phosphoinositolglykan-Peptide nahe der Auftragsstelle auf einer Silikagelplatte (Typ 60) mit Methanol eluiert und anschließend in einem zweiten Lauf in einem basischen System (Chloroform/Methanol/Ammoniak/Wasser 45 : 45 : 3,5: 10) rechromatographiert. Die Phosphoinositolglykan-Peptidderivate werden erneut von der Platte eluiert (R_{f} = 0,45) und mit Chloroform/Methanol (2 : 1) extrahiert. Die organische Phase wird gewaschen und evaporiert und das Material wird in Phosphatpuffer mit 0,5 % TX-100 suspendiert.
e) Die in a) bis d) erhaltenen Phosphoinositolglykane oder Phosphoinositolglykan-Peptide werden mit 10 Units Phosphatidylinositol-spezifischer Phospholipase C, EC 3.1.1.5, (Bacillus cereus; Sigma, Deisenhofen) in 0,2 ml 0,2 M Kaliumphosphat (pH 7,2), 2 mM DTT, 10 mM MgCl₂, 50 mM NaCl, 0,05 % TX-100 für 2 Stunden bei 37 °C inkubiert. Nach Zugabe von 10 mM EDTA werden die Spaltprodukte durch Dünnschichtchromatographie im basischen Laufmittelsystem (siehe d) voneinander getrennt. Material in unmittelbarer Nähe der Auftragstelle wird von der Platte eluiert und mit 2 % Poly(ethylenglycol)₈-Mono-(octylphenylether) (TX-114) versetzt. Durch Erwärmung und Zentrifugation wird eine Phasentrennung eingeleitet. Die wäßrige Phase wird im Speedvac Konzentrator konzentriert.
f) Zur Standardisierung der Phosphoglykan-Peptide wird der Stickstoff des freien Glukosamins von dem Material aus den Verfahrensschritten a) bis d) durch Permethylierung in ein radioaktiv markiertes Trimethylammonium-Kation überführt. Dazu wird die Probe in Vakuum getrocknet und mit 100 µl Dimethylsulfoxid versetzt. Nach Ultraschallbehandlung (1 min) wird 10 mg NaOH und 40 µl [¹²⁵J]Methyljodid (0,2 µCi; NEN-Dupont, Dreieich) zugegeben. Nach Rühren für 45 min bei 25 °C wird das Lösungsmittel in der Speedvac entfernt und 400 µl H₂O hinzugefügt. Die Probe wird dreimal mit Chloroform und die Chloroform-Extrakte dreimal mit H₂O gewaschen. Das Chloroform wird unter N₂ evaporiert. Die Permethylierung erfolgt über einen weiten Konzentrationsbereich linear und quantitativ. In den Testen auf insulinartige Wirkung werden äquivalente Volumina (gleiche dpm-Werte) an Phosphoinositolglykan-Peptiden eingesetzt (1 - 100 "arbitrary units").
g) Inkubation des unter a) bis d) erhaltenen Materials mit salpetriger Säure führt zu Spaltung des Phosphoinositolglykans.

### Beispiel 4

### Strukturmerkmale der Phosphoinositolglykan-Peptide

a) Hefezellen werden wie im Beispiel 1 gezeigt in Anwesenheit von radioaktiv markierten Substraten (Firma NEN-Dupont, Dreieich):
   Stearinsäure
   Myo-Inositol
   Ethanolamin
   Glucosamin oder
   Mannose
   angezogen. Die gesamte Plasmamembranfraktion oder das durch Affinitätschromatographie gereinigte cAMP-Bindeprotein (siehe Beispiel 2) wird einer SDS-Polyacrylamidgelektrophorese unterworfen. Färbung mit Coomassie blue oder durch Autoradiographie der Komponenten zeigt, daß alle obengenannten radioaktiven Komponenten in das Phosphoinositolglykan-Peptid eingebaut werden.
b) Das wie in Beispiel 3 a) erhältliche Phosphoinositolglykan-Peptid wird chemisch und enzymatisch weiter gespalten. Die Spaltprodukte werden durch Dünnschichtchromatographie und Messung der Radioaktivitätsverteilung ([³H]Stearinsäure und [¹⁴C]Myo-Inositol) analysiert. Die verbleibende radioaktiv markierte Struktur wird aus der Platte eluiert und der nächsten Spaltreaktion unterworfen. Aus der nach Pronase-Verdau entstandenen Struktur wird durch Deaminierung mit salpetriger Säure Phosphatidylinositol (PI) freigesetzt. Dieses wird durch Phospholipase D in Phosphatidylsäure oder durch Phospholipase C in Diacylglycerol umgewandelt. Dabei geht die Myo-Inositol-Markierung verloren, die radioaktive Markierung durch Stearinsäure blieb jedoch erhalten. Die Phosphatidylsäure wird anschließend durch Acetolyse in Diglyzeridacetat überführt. Aus dieser Struktur wie auch aus dem Diacylglycerol wird schließlich durch alkalische Hydrolyse Stearinsäure freigesetzt.
c) Die nach Beispiel 3a) und 3e) erhaltene Struktur wird getrocknet und mit HF (60 % in Wasser, 16 Stunden, 0 °C) behandelt und die erhaltenen Oligosaccharide werden mit 2M Trifluoressigsäure hydrolysiert (4 Stunden, 100°C). Anschließend wird die Reaktionslösung getrocknet und die vorhandenen Zucker reduziert (1 % NaBH₄, in 0,1 M Ammoniumhydroxid, 1 Stunde, 37 °C) und abschließend acetyliert (1 : 1 Gemisch aus Pyridin und Essigsäureanhydrid, 1 Stunde, 60 °C). Die Gaschromatographie erfolgte mit einem Gaschromatographen der Firma Packard, Modell 428; Säule 100/120 Supelcoport (Supelco, Bellefonte, USA) bei 60 °C. Es ergab sich fologende qualitative Zusammensetzung:
   Mannose, Galaktose, Myo-Inositol und Glucosamin.
d) Die nach Beispiel 3a) und 3e) erhaltene Struktur wurde wie bei c) mit HF (60 % in Wasser) und 4 M HCl 16 Stunden bei 100 °C hydrolysiert. Eine Trennung auf einem Aminosäureanalysator (Biotronic, LC 6001) ergab folgende Bestandteile:
   Asparaginsäure, NH₃, Ethanolamin und Glucosamin.

In Anbetracht der Hydrolysebedingungen, dem Verhältnis von Asparaginsäure und NH₃, sowie den Ergebnissen nach Beispiel 3a) ist die im nativen Phosphoinositolglykan-Peptid vorliegende Aminosäure Asparagin.

### Beispiel 5

Die biologische Aktivität der erfindungsgemäßen Phosphoinositolglykan-Peptide (PGP) wird an Hand von präparativ isolierten Fettzellen und Diaphragma-Stücken aus der Ratte bestimmt.

Der Begriff "PIG" bedeutet Phosphoinositolglykan ohne Peptid, erhalten nach Beispiel 3 d) und 3 e); "PIG-N" bedeutet Phosphoinositolglykan mit Asparagin, erhalten nach Beispiel 3 a) und 3 e); "PIG-NCY" bedeutet Phosphoinositolglykan mit dem Peptid Asn-Cys-Tyr, erhalten nach Beispiel 3 b) und 3 e); "PIG-NCYE" bedeutet Phosphoinositolglykan mit dem Peptid Asn-Cys-Tyr-Glu, erhalten nach Beispiel 3 c) und 3 e); "PIG-NCY (na)" bedeutet Material erhalten nach Beispiel 3 b) und 3 e), das mit salpetriger Säure gespalten wurde (siehe Beispiel 3 g). Der Begriff "basal" steht für Aktivität ohne Stimulation, Insulin steht für Humaninsulin und dpm steht für radioaktive Zerfälle pro Minute.

Die Präparation von Fettzellen aus der Ratte erfolgte wie folgt:
Fettgewebe des Nebenhodens (Wistar-Ratte, 160 - 180 g, keine Futterbeschränkung) wird mit Kollagenase verdaut und die entstandenen vereinzelten Fettzellen werden durch Flotation mehrmals gewaschen.

Präparation von Diaphragma-Stücken aus der Ratte:
Aus mehrmals gewaschenen Hemi-Diaphragmen (Wistar-Ratte, 60 - 70 g, keine Futterbeschränkung) wurden kleine Gewebsstücke (5 mm Durchmesser) gestanzt.

Zur Inaktivierung des Insulinrezeptors von Ratten-Fettzellen werden die Zellen mit 10 bis 40 µg/ml Trypsin behandelt. Nach Zugabe von Proteaseinhibitoren werden die Zellen zweimal durch Flotation gewaschen und die Inkubation für 15 min bei 37 °C fortgesetzt. Diese Zellen werden dann für den Test auf Stimulation der Lipogenese durch die Phosphoinositolglykan-Peptide benutzt. Eine Kontrollinkubation mit Insulin zeigt, daß die Trypsin-behandelten Zellen nur eine sehr geringe Insulin-stimulierbare Lipogenese und damit nur eine recht begrenzte Zahl an funktionellen Insulinrezeptoren aufweisen.

Zur Inaktivierung des Insulinrezeptors von Rattendiaphragma werden die Gewebsstücke unter ständiger Begasung mit O₂ in KRH-Puffer (0,1 mM Glukose in Gegenwart von 50 µg/ml Phorbolester Tetradecanoylphorbolacetat) für 90 min bei 25 °C inkubiert. Nachfolgend werden die Gewebsstücke zweimal mit KRH-Puffer gewaschen und für die jeweiligen Teste verwendet.

In den nachfolgenden Versuchen werden die Versuchsergebnisse die mit Gewebe oder Zellen erzielt wurden, in denen der Insulinrezeptor inaktiviert wurde, jeweils in Klammern angegeben. In keinem der nachfolgenden Teste zeigt PIG-NCYE eine Wirkung.

### a) Glykogenese

Dieser Test bestimmt die Insulin-stimulierbare Glykogensynthese in Muskelzellen, welche den Glukosetransport über die Plasmamembran und die Umwandlung der Glukose in Glykogen einschließlich der funktionellen Insulin-Signalübertragungskaskade umfaßt.

Diaphragmastücke werden in KRH-Puffer mit 50 µM D-[U-¹⁴C]Glukose in Gegenwart oder Abwesenheit von Insulin und PGP für 15 min bei 37 °C inkubiert. Nach Absaugen des Mediums werden die Gewebestücke intensiv gewaschen, bei -70 °C eingefroren und nachfolgend bei 2 °C in einem Polytron-Zerkleinerer homogenisiert. Das Homogenat wird zentrifugiert (2000 g) und der Überstand auf Filterpapier pipettiert. Zur Bestimmung des gebildeten Glykogens werden die Filter in TCA (5 %) überführt, mit Ethanol und Aceton gewaschen, getrocknet und ihre Radioaktivität über Szintillationsmessung ([¹⁴C]Glycogen [dpm*10⁻³]) bestimmt. Die Einheiten für die Phosphoinositolglykan-Peptide sind arbitrary units, definiert wie in Beispiel 3 f).

Die Tabelle 1 zeigt die Ergebnisse:

**Tabelle 1:**

| Glykogenese [dpm·10⁻³] | | | | | | |
|---|---|---|---|---|---|---|
| | Basal | Insulin | PIG | PIG-N | PIG-NCY | PIG-NCY (na) |
| | | | | | | |
| | 3,3(3,4) | | | | | |
| 10 nM | | 8,4(4,1) | | | | |
| | | | | | | |
| 1 | | | 3,2(3,1) | | | |
| 5 | | | 3,6(3,5) | | | |
| 25 | | | 4,4(3,9) | | | |
| 100 | | | | | | |
| | | | | | | |
| 1 | | | | 3,0(2,5) | | |
| 10 | | | | 4,2(3,8) | | |
| 100 | | | | 6,7(6,2) | | |
| | | | | | | |
| 1 | | | | | 3,2(2,7) | |
| 5 | | | | | 4,8(4,3) | |
| 25 | | | | | 6,5(5,8) | |
| 100 | | | | | 8,0(7,1) | |
| | | | | | | |
| 1 | | | | | | 3,0(2,5) |
| 10 | | | | | | 3,2(2,9) |
| 100 | | | | | | 3,6(3,4) |

### b) Glykogensynthese

Der Test bestimmmt die Insulin-stimulierbare Umwandlung der aktivierten Glukose (UDP-Glukose) in Glykogen (Glykogensynthase-Aktivität) einschließlich einer funktionellen Insulin-Signalübertragungskaskade. Glukose-Transport und Aktivierung der Glukose werden umgangen (im Unterschied zur Messung der Glykogensynthese, siehe a).

Diaphragmastücke werden mit D-Glukose (0,1 mM) in Gegenwart oder Abwesenheit von Insulin und PGP für 30 min bei 37 °C inkubiert. Ein Homogenat wird hergestellt und zentrifugiert (20000 x g). Der Überstand wird mit U-[¹⁴C]UDP-Glukose (0,3 mM) in Gegenwart von entweder 0,1 mM oder 10 mM Glukose-6-Phosphat für 60 min bei 37 °C inkubiert. Die Mischungen werden auf Filterpapier transferiert und die Filter wie oben behandelt. Die Glykogensynthase-Aktivität wird als fraktionale Geschwindigkeit zwischen der I-Form des Enzyms (unabhängig von Glukose-6-Phosphat, dephosphoryliert, entspricht der Menge an aktivem Enzym im Homogenat) und der D-Form des Enzyms (abhängig von Glukose-6-Phosphat, phosphoryliert, entspricht der Gesamtmenge an aktivierbarem Enzym im Homogenat) berechnet ([¹⁴C]Glykogen [dpm*10⁻³]).

Tabelle 2 zeigt die Ergebnisse:

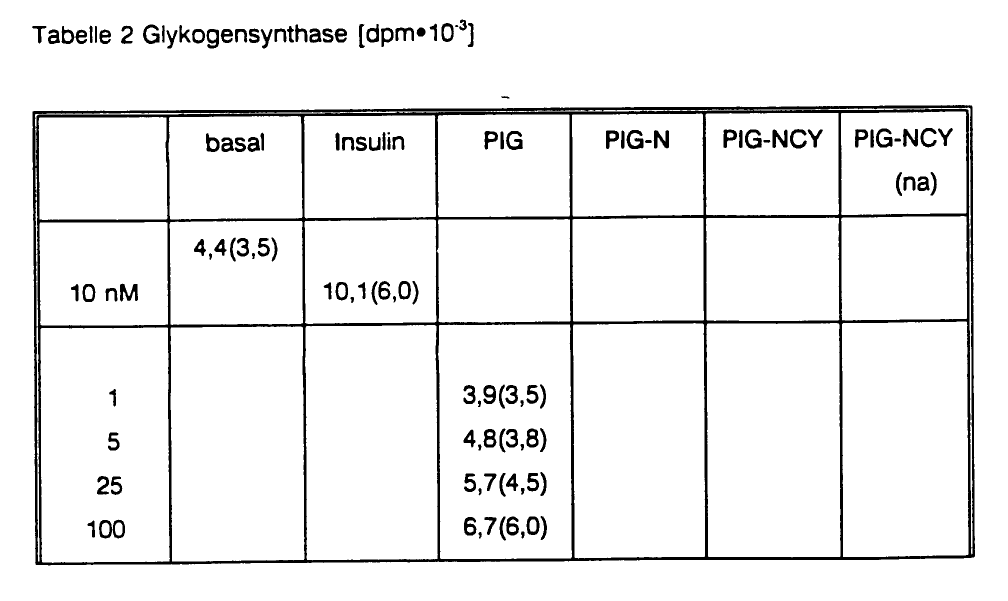

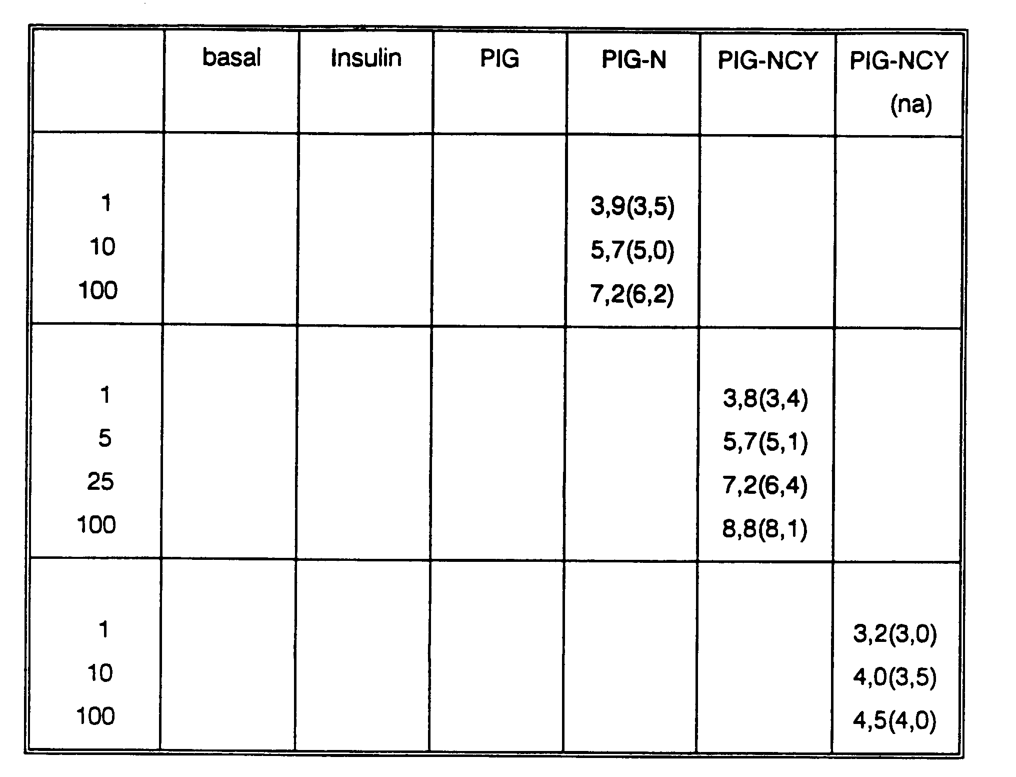

### c) Lipogenese

Dieser Test bestimmt die Insulin-stimulierbare Umwandlung von Glukose in Toluol-lösliche Produkte (Triglyzeride, Phospholipide, Fettsäuren), welche den Glukose-Transport und die Triglyzerid (Glyzerin-3-P-Synthese, Veresterung)/Phospholipid-/Fettsäure-Synthese einschließlich der Insulin-Signalübertragungskaskade erfordert.

Ratten-Fettzellen in KRH-Puffer werden mit D-[3-³H]Glukose (0,2 mM oder 1 mM Endkonz.) in Gegenwart oder Abwesenheit von Insulin und PGP für 90 min bei 37 °C inkubiert. Durch Zugabe eines Toluol-löslichen Szintillationscocktails werden die Zellen aufgeschlossen und die Lipide von wasserlöslichen Produkten und dem Inkubationsmedium abgetrennt. Nach Phasentrennung wird die in Lipide inkorporierte Radioaktivität durch Szintillationsmessung direkt ohne Entfernung der wäßrigen Phase bestimmt ([³H]Lipid [dpm*10⁻³]).

Tabelle 3 zeigt die Ergebnisse:

**Tabelle 3**

| Lipogenese [dpm*10⁻³] | | | | | | |
|---|---|---|---|---|---|---|
| | basal | Insulin | PIG | PIG-N | PIG-NCY | PIG-NCY (na) |
| | 1,0(1,0) | | | | | |
| 10 nM | | 11,2(1,9) | | | | |
| 1 | | | 1,0(0,9) | | | |
| 5 | | | 2,1(1,5) | | | |
| 25 | | | 3,1(3,0) | | | |
| 100 | | | 4,2(3,5) | | | |
| 1 | | | | 1,0(1,2) | | |
| 10 | | | | 3,5(3,3) | | |
| 100 | | | | 5,4(4,8) | | |
| 1 | | | | | 1,0(0,9) | |
| 5 | | | | | 3,5(3,0) | |
| 25 | | | | | 5,5(5,2) | |
| 100 | | | | | 6,8(5,9) | |
| 1 | | | | | | 1,0(0,9) |
| 10 | | | | | | 1,0(1,5) |
| 100 | | | | | | 2,1(2,5) |

### d) Intrinsische Glukosetransportaktivität

Isolierte Plasmamembranvesikel werden aus Rattenfettzellen (siehe Beispiel 5) gewonnen, in dem die Fettzellen zweimal in Homogenisierungspuffer (20 mM Trihydroxyaminomethan (Tris)/HCl, pH 7,4, 1 mM EDTA, 0,25 M Saccharose) gewaschen und dann bei 4°C in 20 ml des gleichen Puffers homogenisiert werden (Glas-Homogenisator mit Teflon-Pistill).

Das Homogenisat wird zentrifugiert (16000 x g, 15 min), das Sediment wird im gleichen Puffer suspendiert und erneut zentrifugiert. Das Sediment wird in 5 ml Homogenisierungspuffer suspendiert und auf ein Saccharose-Kissen (1,12 M Saccharose, 20 mM Tris/HCl, pH 7,4, 1 mM EDTA) geschichtet, nach Zentrifugation (100000 x g, 70 min) wird die Interphase mit den Plasmamembranvesikeln mit einer Spritze entnommen, mit 45 ml Puffer verdünnt und erneut zentrifugiert (48000 x g, 45 min). Das Sediment wird in 10 ml Puffer suspendiert, erneut zentrifugiert und in 3 ml Puffer erneut suspendiert.

Isolierte Plasmamembranvesikel werden in Gegenwart oder Abwesenheit von Insulin/PGP für 30 min bei 25 °C inkubiert. Nachfolgend werden die Vesikel mit 50 µM D-[3-³H]-Glukose und L-[1-¹⁴C]Glukose der gleichen spezifischen Radioaktivität für 90 sec bei 25 °C inkubiert. Die Mischungen werden rasch über Nitrozellulose-Filter abgesaugt. Die Filter werden ausgiebig gewaschen und getrocknet. Ihre Radioaktivität wird durch Flüssigszintillationsmessung bestimmt. Der spezifische Transport (D-[³H]Glucose/L-¹⁴C]Glucose [dpm*10⁻³]) wird als Differenz zwischen der [³H]- und [¹⁴C]-Radioaktivität errechnet.

Tabelle 4 zeigt die Ergebnisse:

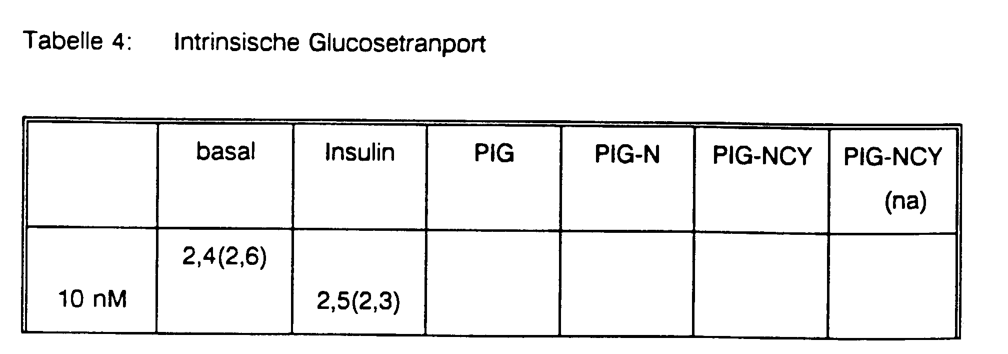

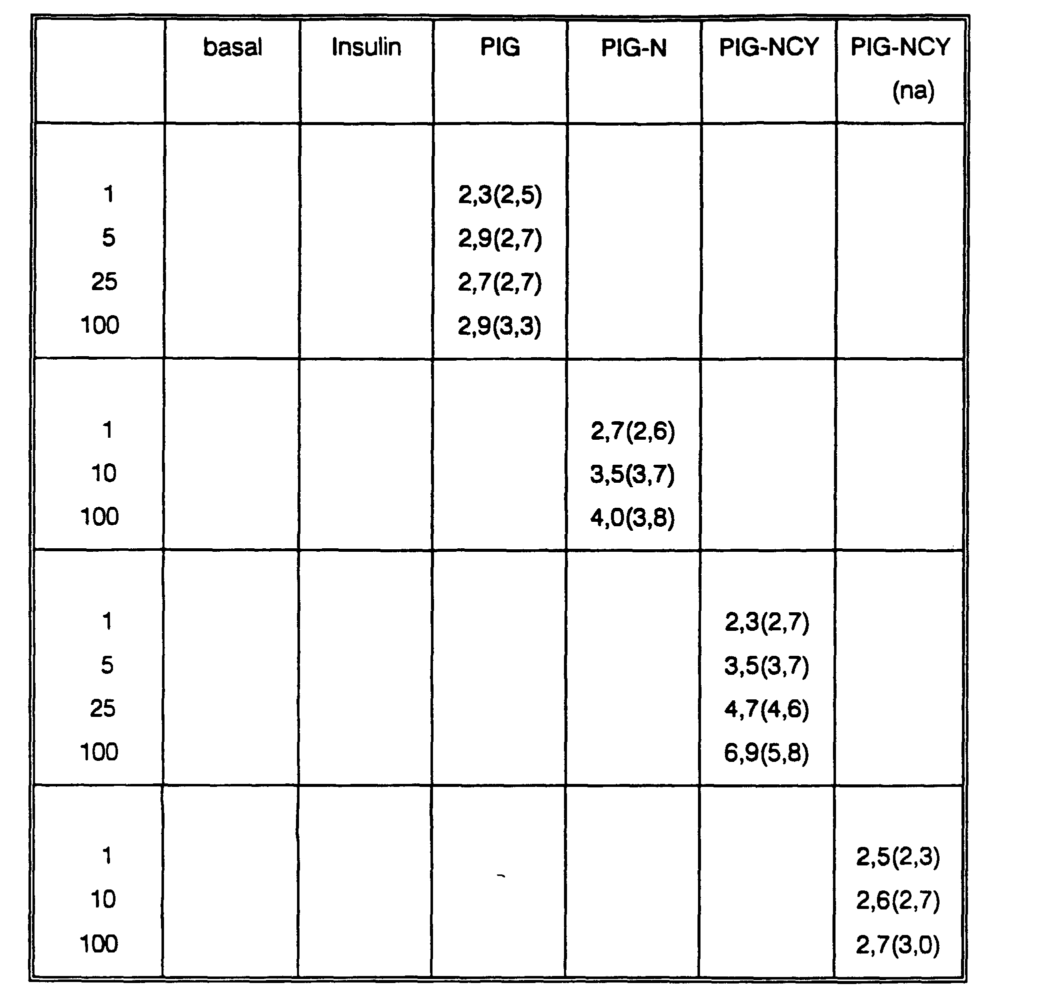

### e) Proteinsynthese

Im Unterschied zu den in den vorherigen Bestimmungen gemessenen metabolischen Insulinwirkungen gehört die Stimulation der Proteinsynthese zu den Langzeitwirkungen von Insulin (als Wachstumshormon). Der Assay schließt die Insulin-Signalkaskade ein.

Ratten-Fettzellen werden in "Dulbecco's modified essential medium" (DMEM), verarmt an Leucin, in Primärkultur mit 50 µM L-[³H]-Leucin in Gegenwart von Insulin und PGP für 4 Stunden bei 37 °C inkubiert. Die Zellen werden durch die Ölzentrifugationstechnik vom umgebenden Medium abgetrennt und mit Wasserkompatiblem Szintillationscocktail versetzt. Nach Zentrifugation wird der Proteinniederschlag mit Aceton gewaschen, in 1 % SDS suspendiert und mit Szintillationscocktail versetzt. Die zellassoziierte Radioaktivität, bestimmt durch Szintillationsmessung dient als Maß für die Proteinsynthese und den Aminosäuretransport durch die Plasmamembran ((³H]Leucin [dpm*10⁻⁴]).

Tabelle 5 zeigt die Ergebnisse:

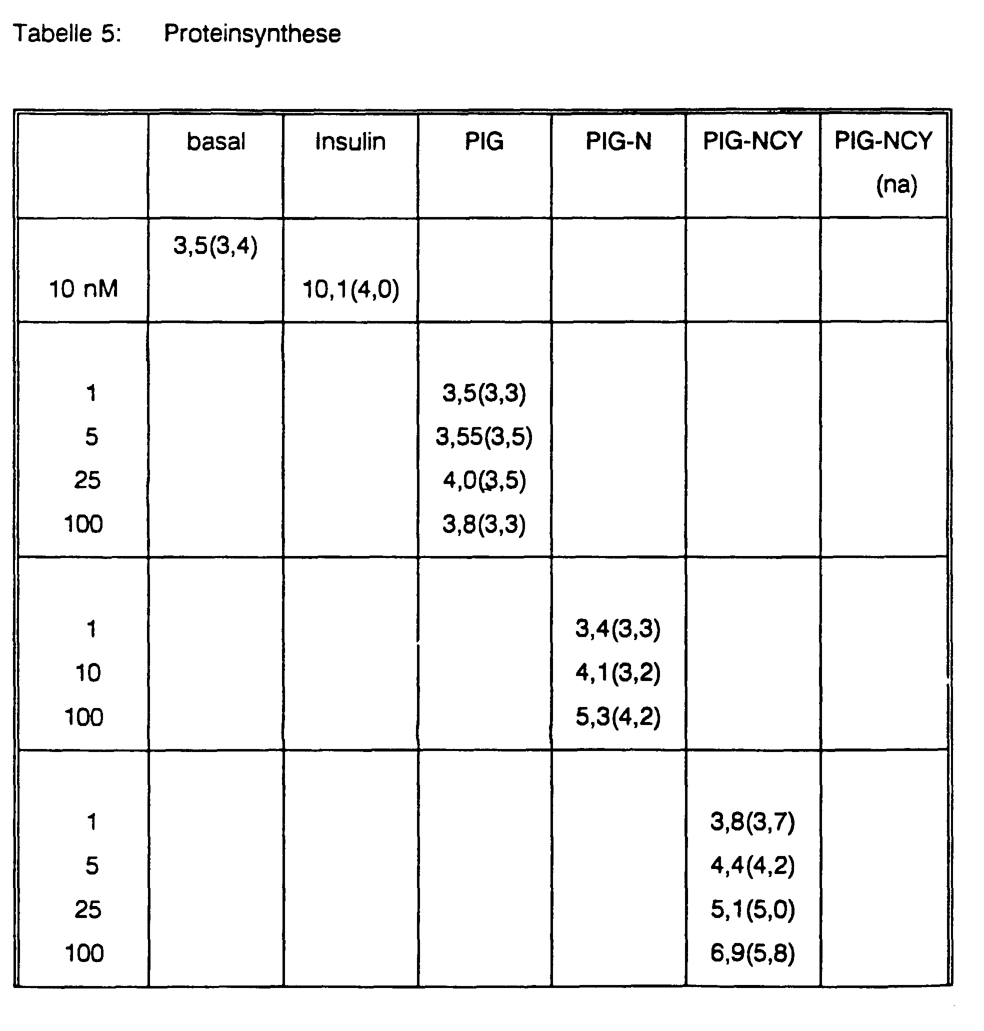

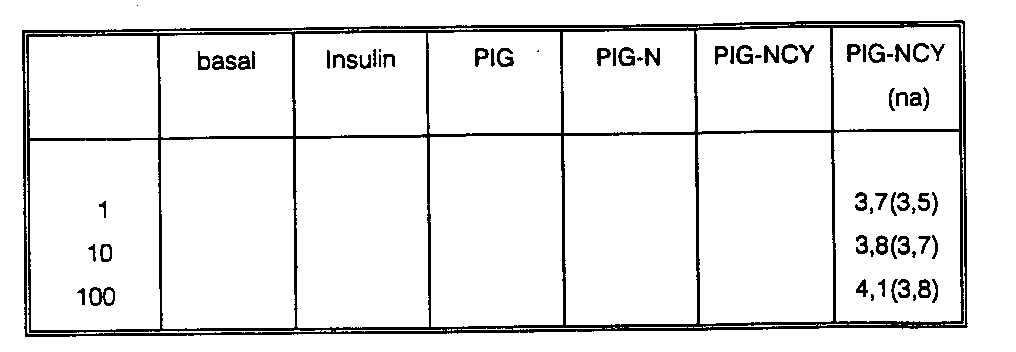

## Patentansprüche

1. Phosphoinositolglykan-Peptid, dadurch gekennzeichnet, daß es Phosphoinositolglykan, Ethanolamin, Tripeptid und/oder physiologisch verträgliche Salze vom Phosphoinositolglykan-Peptid enthält und insulinartige Wirkung besitzt.

2. Phosphoinositolglykan-Peptid nach Anspruch 1, dadurch gekennzeichnet, daß es mindestens einen Glucosamin-, Galaktose-, Mannose-, Myo-lnositol-, Phosphorsäure-, Ethanolaminrest und den Peptidrest mit der Sequenz Asn-Cys-Tyr enthält und insulinartige Wirkung besitzt.

3. Phosphoinositolgtykan-Peptid nach einem oder mehreren der Ansprüche 1 oder 2, dadurch gekennzeichnet, daß es mindestens Phosphoinositolglykan oder Phosphoinositolglykan und Asparagin als Peptidrest enthält und insulinartige Wirkung besitzt.

4. Verfahren zur Herstellung des Phosphoinositolglykan-Peptids nach einem oder mehreren der Ansprüche 1 bis 3, dadurch gekennzeichnet, daß man
a) Organismen, die Adenosin-3',5'-cyclischen-monophosphat-Bindeprotein enthalten, verwendet,
b) das Adenosin-3',5'-cyclischen-monophosphat-Bindeprotein isoliert,
c) den Proteinanteil von Adenosin-3',5'-cyclischen-monophosphat-Bindeprotein abspaltet und das entstandene Glycosyl-phosphatidylinositol-Peptid gegebenenfalls reinigt,
d) von dem im Verfahrensschritt c) erhaltenen Produkt ein Mono- oder Diacylglycerin abspaltet und
e) das in Verfahrensschritt d) entstandene Phosphoinositolglykan-Peptid isoliert und gegebenenfalls zu entsprechenden physiologisch verträglichen Salzen umsetzt.

5. Verfahren nach Anspruch 4, dadurch gekennzeichnet, daß Hefezellen verwendet werden und der Proteinanteil enzymatisch abgespalten wird.

6. Verfahren nach Anspruch 4 oder 5, dadurch gekennzeichnet, daß die Reihenfolge der Verfahrensschritte c) und d) vertauscht wird.

7. Verfahren nach einem oder mehreren der Ansprüche 4 bis 6, dadurch gekennzeichnet, daß Saccharomyces cerevisae DSM 6649, Endoproteinase Glu-C, EC 3.4.21.19 und/oder Phospholipase C, EC 3.1.1.5, verwendet wird.

8. Arzneimittel, gekennzeichnet durch einen wirksamen Gehalt an Phosphoinositolglykan-Peptid -Peptid nach einem oder mehreren der Ansprüche 1 bis 3.

9. Arzneimittel nach Anspruch 8, dadurch gekennzeichnet, daß es ein Insulin, vorzugsweise Rinder-, Schweine- oder Humaninsulin enthält.

10. Verfahren zur Herstellung eines Arzneimittels nach Anspruch 8 oder 9, dadurch gekennzeichnet, daß man mindestens ein Phosphoinositolglykan-Peptid nach einem oder mehreren der Ansprüche 1 bis 3, mit einem physiologisch annehmbaren Träger, sowie gegebenenfalls geeigneten Zusatz-, Wirk- und/oder Hilfsstoffen in eine geeignete Darreichungsform bringt.

11. Verwendung von Phosphoinositolglykan-Peptid nach einem oder mehreren der Ansprüche 1 bis 3 zur Herstellung eines Arzneimittels zur Behandlung von Diabetes mellitus oder nicht insulinabhängigem Diabetes.

## Claims

1. A phosphoinositol-glycan-peptide, which contains phosphoinositol glycan, ethanolamine, tripeptide and/or physiologically tolerated salts of phosphoinositol-glycan-peptide, and has an insulin-like action.

2. A phosphoinositol-glycan-peptide as claimed in claim 1, which contains at least one glucosamine, galactose, mannose, myo-inositol, phosphoric acid, ethanolamine residue and the peptide residue with the sequence Asn-Cys-Tyr, and has an insulin-like action.

3. A phosphoinositol-glycan-peptide as claimed in one or more of claims 1 or 2, which contains at least phosphoinositol glycan or phosphoinositol glycan and asparagine as peptide residue, and has an insulin-like action.

4. A process for the preparation of the phosphoinositol-glycan-peptide as claimed in one or more of claims 1 to 3, which comprises
a) using organisms which contain adenosine 3',5'-cyclic monophosphate-binding protein,
b) isolating the adenosine 3',5'-cyclic monophosphate-binding protein,
c) eliminating the protein portion from adenosine 3',5'-cyclic monophosphate-binding protein, and purifying the resulting glycosylphosphatidylinositol-peptide where appropriate,
d) eliminating a mono- or diacylglycerol from the product obtained in process step c) and
e) isolating the phosphoinositol-glycan-peptide produced in process step d), and converting into corresponding physiologically tolerated salts where appropriate.

5. The process as claimed in claim 4, wherein yeast cells are used, and the protein portion is eliminated enzymatically.

6. The process as claimed in claim 4 or 5, wherein the sequence of process steps c) and d) is reversed.

7. The process as claimed in one or more of claims 4 to 6, wherein Saccharomyces cerevisae DSM 6649, endoproteinase Glu-C, EC 3.4.21.19 and/or phospholipase C, EC 3.1.1.5, is used.

8. A pharmaceutical which has an effective content of phosphoinositol-glycan-peptide as claimed in one or more of claims 1 to 3.

9. A pharmaceutical as claimed in claim 8, which contains an insulin, preferably bovine, porcine or human insulin.

10. A process for the preparation of a pharmaceutical as claimed in claim 8 or 9, which comprises converting at least one phosphoinositol-glycan-peptide as claimed in one or more of claims 1 to 3 with a physiologically acceptable vehicle and, where appropriate, suitable additives, active substances and/or ancillary substances into a suitable dosage form.

11. The use of phosphoinositol-glycan-peptide as claimed in one or more of claims 1 to 3 for preparing a pharmaceutical for the treatment of diabetes mellitus or non-insulin-dependent diabetes.

## Revendications

1. Phospho-inositol-glycane-peptide, caractérisé en ce qu'il comprend du phospho-inositol-glycane, de l'éthanolamine, un tripeptide et/ou des sels physiologiquement acceptables du phospho-inositol-glycane-peptide et possède une activité de type insuline.

2. Phospho-inositol-glycane-peptide selon la revendication 1, caractérisé en ce qu'il comprend au moins un motif glucosamine, galactose, mannose, myo-inositol, acide phosphorique, éthanolamine et le motif peptidique avec la séquence Asn-Cys-Tyr et possède une activité de type insuline.

3. Phospho-inositol-glycane-peptide selon la revendication 1 ou 2, caractérisé en ce qu'il comprend au moins un phospho-inositol-glycane ou un phospho-inositol-glycane et l'asparagine comme motif peptidique et qu'il possède une activité de type insuline.

4. Procédé pour la préparation du phospho-inositol-glycane-peptide selon une ou plusieurs des revendications 1 à 3, caractérisé en ce que
a) on utilise des organismes qui contiennent des adénosine-3',5'-cyclique monophosphate-protéines liantes,
b) on isole l'adénosine-3',5'-cyclique monophosphate-protéine liante
c) on élimine la partie protéinique de l'adénosine-3',5'-cyclique monophosphate-protéine liante et on purifie éventuellement le glycosyl-phosphatidylinositol-peptide,
d) on élimine du produit obtenu à l'étape c) une mono ou diacylglycérine et
e) on isole le phospho-inositol-glycane-peptide obtenu à l'étape d) et on le transforme éventuellement en un sel physiologiquement acceptable correspondant.

5. Procédé selon la revendication 4, caractérisé en ce que l'on utilise des cellules de levure et que l'on élimine la partie protéinique à l'aide d'une enzyme.

6. Procédé selon la revendication 4 ou 5, caractérisé en ce que l'ordre des étapes c) et d) est inversé.

7. Procédé selon une ou plusieurs des revendications 4 à 6, caractérisé en ce que l'on utilise *Saccharomyces cerevisae* DSM 6649, l'endoprotéinase Glu-C, EC 3.4.21.19 et/ou la phospholipase C, EC 3.1.1.5.

8. Médicament, caractérisé par une teneur active en phospho-inositol-glycane-peptide selon une ou plusieurs des revendications 1 à 3.

9. Médicament selon la revendication 8, caractérisé en ce qu'il contient une insuline, de préférence l'insuline de boeuf, de porc ou l'insuline humaine.

10. Procédé pour la préparation du médicament selon la revendication 8 ou 9, caractérisé en ce que l'on met sous une forme de présentation appropriée au moins un phospho-inositol-glycane-peptide selon une ou plusieurs des revendications 1 à 3, avec un véhicule physiologiquement acceptable ainsi qu'éventuellement des additifs, principes actifs et/ou adjuvants appropriés.

11. Utilisation de phospho-inositol-glycane-peptides selon une ou plusieurs des revendications 1 à 3 pour la préparation d'un médicament pour le traitement du diabète sucré ou du diabète non insulino-dépendant.
